# EUROPÄISCHE PATENTSCHRIFT

(11) **EP 0 653 210 B1**
(45) Veröffentlichungstag und Bekanntmachung des Hinweises auf die Patenterteilung: **19.03.1997**
(21) Anmeldenummer: 94113875.2
(22) Anmeldetag: 05.09.1994
(51) Int. Cl.: A61K 31/505, A61K 31/635, A61K 9/08, A61K 47/12, A61K 47/18

(54) **Intravenös verabreichbare wässrige Lösung von Sulfametrol und Trimethoprim**
Intravenous applicable aqueous solution compressing sulfametrol and trimethoprim
Solution aqueuse pour l'administrations intraveineuse contenant de sulfametrol et triméthoprim

(30) Priorität: 14.09.1993 DE 4331147
(43) Veröffentlichungstag der Anmeldung: 17.05.1995
(73) Patentinhaber: Nycomed Austria GmbH, 4020 Linz (AT)
(72) Erfinder: Groke, Karl, Dr., A-8063 Eggersdorf (AT)
(74) Vertreter: Plougmann, Vingtoft & Partners A/S

(56) Entgegenhaltungen:
- EP-A- 0 037 501
- AT-A- 270 869
- DE-A- 2 400 218
- DE-A- 2 731 013
- US-A- 5 008 255

## Beschreibung

Die vorliegende Erfindung betrifft eine intravenös verabreichbare, wäßrige Lösung des Sulfonamids 3-Methoxy-4-(4'-benzolsulfonamido)-1,2,5-thiadiazol (Sulfametrol) und des Sulfonamidpotentiators 2,4-Diamino-5-(3',4',5'-trimethoxy-benzyl)-pyrimidin (Trimethoprim), die einen pH-Wert im Bereich von 6,4-7,2 besitzt, auf übliche Weise sterilisiert werden kann und sich durch eine gute Lagerstabilität auszeichnet.

Es ist bekannt, daß die in der Therapie gut eingeführten Kombinationen von Sulfonamiden mit dem Sulfonamidpotentiator Trimethoprim bei der Überführung in parenteral verträgliche Lösungen große Schwierigkeiten bereiten. Diese waren vor allem darauf zurückzuführen, daß die für solche Kombinationen eingesetzten Sulfonamide, wie unter anderem das Sulfametrol im alkalischen Milieu, z.B. im Falle des Sulfametrols bei pH-Werten von 7,4 und höher, zwar gut wasserlösliche Salze, z.B. Na-Salze, bilden, die schwache Base Trimethoprim aber im alkalischen Bereich bereits als freie Base vorliegt und in dieser Form in Wasser nicht löslich ist. Im sauren Bereich geht hingegen das Trimethoprim unter Bildung von Säureadditionssalzen in Lösung, die in Frage kommenden Sulfonamide sind jedoch bei den dazu erforderlichen pH-Werten in Wasser nicht löslich. Es stand daher nur ein Ausweg offen, für einen der beiden Bestandteile der Kombination zur Erzielung einer Lösung ein organisches Lösungsmittel einzusetzen. (AT-PS 270.869, DE-A 2 400 218).

Aus der AT-PS 270.869 sind gut verträgliche, intravenös verabreichbare, wäßrige Lösungen aus Sulfametrol und Trimethoprim mit einem pH-Wert von 7,4 bekannt geworden, in denen das Sulfametrol als Salz mit einem starken Gegenelektrolyten, insbesondere NaOH, eingesetzt wird, während das Trimethoprim durch Zugabe von Zuckeralkoholen, wie Sorbit, Xylit oder Mannit, oder von reduzierenden Zuckern, wie Fructose oder Glucose, als Lösungsvermittler in Lösung gebracht wird.

Eine Ausführungsform dieser Lösungen mit einem pH-Wert von 7,4 und mit dem Zuckeralkohol Sorbit als Lösungsvermittler fand in der Praxis verbreitete Anwendung.

Durch die Situation, daß Zuckeralkohole und Fructose in manchen Ländern in parenteral verabreichbaren Lösungen nicht mehr eingesetzt werden dürfen, wodurch auch der Einsatz dieser Stoffe als Lösungsvermittler betroffen ist, und durch die Schwierigkeit, die das Sterilisieren von Lösungen mit Trimethoprim und Glucose bereitet, stellte sich die Aufgabe, für Trimethoprim einen neuen, physiologisch tragbaren Lösungsvermittler zu finden, der die Bereitung wäßriger, leicht alkalisch reagierender, das Sulfametrol als Alkalisalz gelöst enthaltender Lösungen der Kombination Sulfametrol-Trimethoprim ermöglicht.

Im Rahmen von diesbezüglichen Untersuchungen konnte gefunden werden, daß verschiedene α-Hydroxymonocarbonsäuren- und -dicarbonsäuren sowie die Aminosäuren L-Serin und L-Threonin lösungsvermittelnde Eigenschaften im leicht alkalischen Milieu von pH 7,5 und mehr entfalten. Die damit erzeugten klaren Lösungen erwiesen sich aber nur als metastabil, nach für die Praxis zu kurzen Lagerzeiten konnte ein Auskristallisieren von Trimethoprim registriert werden.

Überraschenderweise konnte nun gefunden werden, daß derartige Lösungen in einem sehr beschränkten, schwach sauren bis neutralen pH-Bereich, nämlich von 6,4 bis 7,2, stabil sind und weder Fällungen oder Kristallisationen von Sulfametrol, noch von Trimethoprim zu verzeichnen sind, wenn als Lösungsvermittler Äpfelsäure, Serin oder Threonin oder Gemische dieser Säuren dienen. Wird dieser pH-Bereich verlassen, werden nur metastabile Lösungen erhalten, die bei pH-Werten über 7,2 Ausfällungen von Trimethoprim, unter 6,4 solche von Sulfametrol aufweisen.

Gegenstand der vorliegenden Erfindung ist demnach eine intravenös verabreichbare, wäßrige Lösung des als Salz vorliegenden Sulfonamids Sulfametrol und von Trimethoprim, die dadurch gekennzeichnet ist, daß sie als Lösungsvermittler für das Trimethoprim Äpfelsäure, L-Serin und/oder L-Threonin, zusammen mit einer starken, physiologisch tolerierbaren, mit dem Sulfonamid und der Äpfelsäure wasserlösliche Salze bildenden Base in einer der vorliegenden Menge des Sulfonamids äquimolaren Menge sowie in einer Menge von etwa 2 Mol pro Mol vorliegender Äpfelsäure enthält, wobei der pH-Wert der Lösung auf den Bereich 6,4-7,2 eingestellt ist.

Während man gemäß bisheriger Überlegung in erster Linie bestrebt war, der Lösung genügend Alkali einzuverleiben, um sicher zu stellen, daß pro Mol Sulfametrol mindestens 1 Mol an der starken Gegenbase zur Verfügung stand und daher ein deutlich im alkalischen Bereich liegender pH-Wert bevorzugt wurde, wird gemäß vorliegender Erfindung maximal ein pH-Wert von 7,2 toleriert. Bevorzugt ist sogar, daß der pH-Wert den Neutralpunkt nicht überschreitet, sondern vielmehr im schwach sauren Bereich bleibt. Der vorzugsweise einzustellende pH-Wert liegt bei 6,5-7,0.

Unter den lösungsvermittelnd wirkenden Säuren gemäß vorliegender Erfindung ist dabei der Äpfelsäure der Vorzug zu geben, da bei dieser Ausführungsform der erfindungsgemäßen Lösung nicht nur jene Menge an Gegenbase, vorzugsweise NaOH, zugegen ist, die der vorhandenen Menge an Sulfametrol äquivalent ist. Es bedarf vielmehr überdies einer weiteren Menge an Gegenbase, die etwa der vorliegenden Menge an Äpfelsäure äquivalent ist. Da hierbei im Vordergrund steht, daß die pH-Grenze von 7,2, vorzugsweise 7,0, nicht überschritten werden darf, bedeutet dies, daß die Menge der Gegenbase gegenüber der Äpfelsäure im geringen Überschuß zu bleiben hat. Daß dabei trotzdem keine Instabilität des Salzes des Sulfametrols bei diesem leicht sauren pH, die durch einen Alkalientzug durch die Äpfelsäure zutage treten würde, auftritt, ist darauf zurückzuführen, daß die zweite Dissoziationskonstante von 1,39 x 10⁻⁵ schwächer als die zweite Dissoziationsstufe von Sulfametrol (Dissoziationskonstante 1,6 x 10⁻⁵) ist und das vorliegende Salz der Äpfelsäure auch dann, wenn seine zweite Stufe nicht voll neutralisiert ist, eine Alkalireserve für das Sulfametrol darstellt, die der Lösung Stabilität verleiht.

Dieser Effekt wird bei Säuren mit stärkerer zweiter Dissoziationsstufe nicht erzielt. Die Folge wäre dann ein Alkalientzug beim Sulfametrol und eine damit verbundene Instabilität, die man nach bisheriger Lehrmeinung durch einen stärker alkalischen pH-Wert der Lösung vermieden hat, der jedoch zu Instabilitäten beim Trimethoprim führt.

Werden die Aminosäuren L-Serin und/oder L-Threonin als Lösungsvermittler eingesetzt, besteht zwar diese Alkalireserve nicht, die Gefahr eines Basenentzuges durch diese Lösungsvermittler ist aber ebenfalls nicht zu erwarten, da sie Ampholyte sind und zur Ionisierung der Carboxylgruppe keiner Gegenbase bedürfen. In diesem Fall benötigt die Lösung nur die dem Sulfametrol äquivalente Menge an Gegenbase und einen geringen Überschuß an dieser Base zur Einstellung des pH-Wertes, je nachdem, wie dieser innerhalb des erfindungsgemäßen Bereiches gewählt wird.

Die lösungsvermittelnde Wirkung der drei erfindungsgemäß eingesetzten Säuren auf das Trimethoprim ist speziell im erfindungsgemäßen Bereich sehr ausgeprägt. Um sicher eine auch über längere Zeit lagerstabile Lösung zu erhalten, empfiehlt es sich, in dieser einen Gehalt von 100 Milliäquivalenten des Lösungsvermittlers pro Liter Lösung einzustellen, das sind bei Äpfelsäure 50 mmol/l, bei L-Serin und L-Threonin 100 mmol/l. Diese Konzentration stellt jedoch nur eine Mindestmenge dar, die ohne Probleme erhöht werden kann und auch soll, um optimale Ergebnisse zu erzielen. Eine Obergrenze ist im Falle des Einsatzes von Äpfelsäure, abgesehen von wirtschaftlichen Überlegungen, jedoch dadurch gegeben, daß der Gehalt an der als Gegenion eingesetzten starken Base in der Tagesdosis in physiologisch vertretbaren Grenzen bleiben muß. So ist z.B. der Tagesbedarf eines Patienten an Natrium 240 mmol, eine Menge, die aus physiologischen Gründen nicht wesentlich überschritten werden sollte, wodurch sich für den Einsatz der Äpfelsäure, wenn sie mit NaOH neutralisiert wird, eine Obergrenze ergibt. Da jedoch die lösungsvermittelnden Eigenschaften der Äpfelsäure bereits bei Konzentrationen knapp über der als zweckmäßig genannten Mindestkonzentration optimal sind, besteht in der Praxis keine Gefahr, daß eine Überschreitung der tolerierbaren Natriummenge eintreten könnte. Beim Kalium-Ion liegt die physiologisch tolerierbare Tagesdosis hingegen so tief, daß eine Verwendung von KOH allein als Gegenbase für Äpfelsäure nicht in Betracht zu ziehen ist.

In der Regel sollten 400 mmol Na-Ion pro Liter der erfindungsgemäßen Lösung nicht überschritten werden, um eine ausreichend rasche Infusionsgeschwindigkeit zu ermöglichen. Diese Menge entspricht dem Einsatz von etwa 190 mmol Äpfelsäure. Derartig hohe Mengen sind zur Erzielung eines optimalen Effektes jedoch gar nicht nötig. Die bevorzugte Menge an L-Äpfelsäure liegt bei 120-180 Milliäquivalenten bzw. 60-90 mmol/Liter, wobei der L-Äpfelsäure aus physiologischen Gründen der Vorzug zu geben ist.

Dienen hingegen die Aminosäuren Serin und/oder Threonin als Lösungsvermittler, ist die benötigte Menge an Gegenbase, wie schon oben gesagt, sehr viel geringer. Auch in diesem Fall hat sich zur Neutralisation, hier nur des Sulfametrols, NaOH sehr bewährt, es ist aber auch möglich KOH allein einzusetzen, da die Konzentration des Sulfametrols pro Liter der Lösung in der Praxis so niedrig gehalten ist, daß eine Überschreitung der tolerierbaren Menge an Kalium-Ion nicht gegeben ist. Bei der bisher in der Praxis eingesetzten Lösung beträgt die Konzentration an Sulfametrol 11,17 mmol/Liter, die des Trimethoprim 2,2 mmol/Liter.

Bei Einsatz dieser beiden Aminosäuren wäre also in Hinblick auf die zwangsläufig damit zu verabreichende Menge an Na- oder K-Ion eine obere Konzentrationsgrenze nicht gegeben. Es ist allerdings zu beachten, daß zu hohe Aufwandmengen an diesen beiden Aminosäuren, die aus technischen Gründen gar nicht erforderlich sind, zu Aminosäureimbalancen führen könnten, vor allem dann, wenn nur eine der beiden Aminosäuren allein eingesetzt wird. Es kann daher von Vorteil sein, diese Säuren im Gemisch, z.B. in einem Molverhältnis von Serin zu Threonin von 1 : 1 bis 2 : 1 einzusetzen, wobei ein Molverhältnis von 2 : 1 bevorzugt ist. Aus dem gleichen Grund kann es auch von Vorteil sein, eine der beiden Aminosäuren mit Äpfelsäure zu kombinieren, wodurch Aminosäureimbalancen nicht zu befürchten sind und auch die Natriumbelastung gegenüber dem Einsatz von Äpfelsäure allein vermindert werden kann.
Da Äpfelsäure, Serin und Threonin der gleichen Aufwandmenge an Milliäquivalenten bedürfen, bleibt diese unabhängig davon, ob Einzelsäuren oder Säuregemische eingesetzt werden, gleich.

Aus physiologischen Gründen sollten die erfindungsgemäßen Lösungen, die ja intravenös verabreicht werden, etwa isoton sein, zumindest aber 2/3 der Isotonie besitzen. Bei Verwendung von Äpfelsäure als Lösungsvermittler werden bei Dosen, die deutlich über der Mindestdosis liegen, aufgrund der Notwendigkeit, äquivalente Mengen eines Gegenions einzusetzen, ohne Zutun bereits Lösungen erhalten, deren Osmolarität entspricht. Bei Dosen in der Nähe der Mindestdosis wird eine Osmolarität von etwa 200 mosmol/l jedoch nicht erreicht, sodaß der Zusatz osmotisch wirksamer, inerter Bestandteile angezeigt ist. Ebenso reicht die sich einstellende Osmolarität bei erfindungsgemäßen Lösungen, für deren Bereitung L-Serin und/oder L-Threonin dienen, nicht aus, um diese Lösungen unbedenklich intravenös verabreichen zu können. Als inerte Substanz zur Einstellung der Osmolarität hat sich vor allem Glycerin bewährt. Vor allem bei Lösungen, die die Amino-hydroxycarbonsäuren enthalten, kann die Osmolarität auch durch Zugabe von Kochsalz auf physiologisch vertretbare Werte eingestellt werden, da solche Lösungen keine hohe Belastung an Natrium besitzen. Bei Einsatz von Äpfelsäure als Lösungsvermittler ist bei Verwendung von Kochsalz zum Zweck der Einstellung der Osmolarität die Gesamt-Na-Belastung im Auge zu behalten.

Zur Herstellung der erfindungsgemäßen Lösung wird zunächst in einer etwas weniger als dem Endvolumen entsprechenden, vorgelegten Wassermenge das Monosalz des Sulfametrols mit einer starken, physiologisch tolerierbaren Base bereitet, der dann entweder Äpfelsäure, zusammen mit etwa der zweifachen Menge ebenfalls einer starken, physiologisch tolerierbaren Base oder aber L-Serin und/oder L-Threonin zugesetzt wird. In die resultierende, klare Lösung wird das Trimethoprim eingetragen. Eine Auflösung desselben ist schon bei Raumtemperatur möglich, zur Beschleunigung empfiehlt es sich jedoch, bei erhöhter Temperatur, die bis zu etwa 90° C betragen kann, zu arbeiten, wobei eine Temperatur von 70° C bis 80° C zu bevorzugen ist. In der so anfallenden Lösung muß dann der pH-Wert auf den gewünschten Wert innerhalb des Bereiches von 6,4 bis 7,2 eingestellt werden. Dabei dient zur Erhöhung des pH-Wertes die Zugabe von NaOH und/oder KOH, bevorzugt NaOH. Sollte sich eine Absenkung des pH-Wertes als erforderlich erweisen, ist es empfehlenswert, als Säure Äpfelsäure einzusetzen. Die Verwendung starker Mineralsäuren, wie etwa Salzsäure könnte zu einer Instabilität der Lösung führen.

Besonders bevorzugt ist es, bei der Herstellung der erfindungsgemäßen Lösung etwas weniger als die äquivalente Menge an der starken Gegenbase einzusetzen, wobei der Unterschuß zweckmäßig so zu wählen ist, daß der pH-Wert in jedem Fall über 6, vorzugsweise bei etwa 6,2 bis 6,3 zu liegen kommt. Nach Auflösung des Trimethoprims wird dann, am besten mit NaOH, die Feineinstellung des pH-Wertes vorgenommen. Wenn erforderlich, werden dann Zusätze zur Anhebung der Osmolarität beigefügt, worauf dann mit Wasser ad infundi das Endvolumen der Lösung eingestellt wird. Ein besonderer Vorteil der erfindungsgemäßen Lösung ist, daß sie bei üblichen Temperaturen, z.B. bei 115° C, im Autoklaven sterilisiert werden können. Sie bleiben bei Lagerung sowohl bei 4° C als auch bei 10° C längere Zeit stabil.

Bei der Wahl des pH-Wertes innerhalb des Bereiches von 6,4-7,2 im Zuge der Herstellung der erfindungsgemäßen Lösung ist zu berücksichtigen, daß bei der Sterilisation leichte pH-Verschiebungen zu höheren Werten auftreten können. Diese Verschiebungen betragen, wenn von einem pH unter 7 ausgegangen wird, nur einige Hundertstel, werden aber größer, wenn der Ausgangs-pH über 7 liegt. So wurden z.B. bei einem Ausgangs-pH von 7,17 Erhöhungen um etwa 1 Zehntel beobachtet, was eine Überschreitung der Obergrenze von 7,2 darstellt. Aus diesem Grund empfiehlt es sich, den pH-Wert vor der Sterilisation so einzustellen, daß eine solche Überschreitung während der Sterilisation auszuschließen ist.

Die erfindungsgemäße Lösung wird zur Bekämpfung von Infektionen eingesetzt, wobei die Tagesdosis vorteilhaft 1,6 g Sulfametrol und 320 mg Trimethoprim, gelöst in 0,5 l Wasser, beträgt. Bevorzugt ist eine Lösung, die pro Liter 11,17 mmol eines Na-Salzes von Sulfametrol, 2,20 mmol Trimethoprim, 140-180 Milliäquivalente L-Äpfelsäure, L-Serin oder L-Threonin oder die gleiche äquivalente Menge an Gemischen derselben, sowie die zur Einstellung des pH-Wertes auf einen Wert von 6,5-7,0 erforderliche Menge an NaOH, gegebenenfalls neben inerten Zusätzen zur Einstellung der Osmolarität auf physiologisch tolerierbare Werte, enthält.

### Beispiel 1:

11,17 mmol = 3,200 g Sulfametrol und 11,17 mmol = 0,447 g NaOH werden in etwa 900 ml Wasser ad infundi zu einer klaren Lösung gelöst. 70 mmol = 9,38 g L-Äpfelsäure werden zusammen mit 5,5 g NaOH zugesetzt und aufgelöst. In die so erhaltene klare Lösung werden 640 mg Trimethoprim unter Erwärmen auf 75° C aufgelöst. Nach dem Abkühlen werden 5 g Glyzerin zur Anhebung der Osmolarität beigefügt, und die Lösung wird mit Wasser ad infundi auf ein Volumen von etwa 995 ml gebracht. Der pH-Wert der Lösung beträgt dann 6,25. Er wird durch Zugabe von 3,05 ml 1 m NaOH auf 7,09 angehoben, worauf dann die Lösung nach Auffüllen auf das Endvolumen auf übliche Weise sterilisiert wird.

Sie besitzt eine Osmolarität von 228 mosmol/l und zeigt bei Lagerung bei 4° C über 4 Monate keinerlei Kristallisationsneigung.

### Beispiel 2:

Sulfametrol, Äpfelsäure, NaOH und Trimethoprim werden, wie in Beispiel 1 beschrieben, zu einer Lösung verarbeitet, nur mit dem Unterschied, daß anstelle von 5,50 g NaOH 5,603 g NaOH zur Neutralisation der Apfelsäure zugesetzt werden. Der pH-Wert der Lösung beträgt nach Zugabe von 5 g Glycerin und Auffüllen bis 995 ml 7,64. Er wird durch Zugabe von 1,1 mmol Äpfelsäure auf 6,64 abgesenkt, worauf die Lösung, wie in Beispiel 1 beschrieben, endgefertigt wird.

### Beispiel 3:

Es wird wie in Beispiel 1 beschrieben, eine Lösung hergestellt, nur mit dem Unterschied, daß zur Feineinstellung des ph-Wertes 0,9 ml 1 m NaOH verwendet werden. Es ergibt sich nach Auffüllen auf 1000 ml ein ph-Wert von 6,47. Diese Lösung wird, wie in Beispiel 1 beschrieben, endgefertigt. Sie zeigt bei Lagerung über 4 Monate keinerlei Kristallisationserscheinungen.

### Beispiel 4:

11,17 mmol = 3,20 g Sulfametrol werden unter Zusatz von 11,50 mmol = 0,46 g NaOH in 900 ml Wasser in Lösung gebracht. In diese Lösung werden 80 mmol = 8,4 g L-Serin und 40 mmol = 4,76 L-Theronin eingetragen und gelöst. In dieser Lösung werden 640 mg Trimethoprim suspendiert und unter Erwärmen auf 70° C aufgelöst. Der pH-Wert der so resultierenden Lösung beträgt 7,03, er wird nicht korrigiert. Nach Zusatz von 65 mmol NaCl zur Einstellung der Osmolarität wird die Lösung auf das Endvolumen von 1000 ml aufgefüllt und auf übliche Weise sterilisiert.

Die resultierende Lösung besitzt eine Osmolarität von 275 mosmol/l. Sie ist bei Lagerung bei 4° C über 4 Monate Lagerzeit stabil.

## Patentansprüche

1. Intravenös verabreichbare, wäßrige Lösung des als Salz vorliegenden Sulfonamides 3-Methoxy-4-(4'-aminobenzolsulfonamido)-1,2,5-thiadiazols und von 2,4-Diamino-5-(3',4',5'-trimethoxybenzyl)-pyrimidin, dadurch gekennzeichnet, daß sie als Lösungsvermittler für das 2,4-Diamino-5-(3',4',5'-trimethoxybenzyl)-pyrimidin Äpfelsäure, L-Serin und/oder L-Threonin, zusammen mit einer starken, physiologisch tolerierbaren, mit dem Sulfonamid und der Apfelsäure wasserlösliche Salze bildenden Base in einer der vorliegenden Menge des Sulfonamids äquimolaren Menge sowie in einer Menge von etwa 2 Mol pro Mol vorliegender Äpfelsäure enthält, wobei der pH-Wert der Lösung auf den Bereich 6,4-7,2 eingestellt ist.

2. Lösung gemäß Anspruch 1, dadurch gekennzeichnet, daß die als Lösungsvermittler dienenden Säuren in einer Menge von mindestens 100 Milliäquivalenten pro Liter Lösung zugegen sind.

3. Lösung gemäß den Ansprüchen 1 oder 2, dadurch gekennzeichnet, daß die als Lösungsvermittler dienenden Säuren in einer Menge von 120-180 Milliäquivalenten pro Liter Lösung zugegen sind.

4. Lösung gemäß den Ansprüchen 1 bis 3, dadurch gekennzeichnet, daß der pH-Wert auf den Bereich von 6,5-7,0 eingestellt ist.

5. Lösung gemäß den Ansprüchen 1 bis 4, dadurch gekennzeichnet, daß der Lösungsvermittler Äpfelsäure und die starke Base NaOH oder ein Gemisch aus NaOH und KOH ist, wobei die Konzentration an KOH 25 mmol/l der Lösung nicht überschreitet.

6. Lösung gemäß den Ansprüchen 1 bis 4, dadurch gekennzeichnet, daß der Lösungsvermittler L-Serin und/oder L-Threonin und die starke Base NaOH und/oder KOH ist.

7. Lösung gemäß den Ansprüchen 1 bis 6, dadurch gekennzeichnet, daß sie eine Osmolarität von mindestens 200 mosmol/l besitzt.

8. Lösung gemäß den Ansprüchen 1 bis 7, dadurch gekennzeichnet, daß der Lösungsvemittler L-Äpfelsäure in Salzform ist.

9. Lösung mit antibakterieller Wirkung gemäß den Ansprüchen 1 bis 8, dadurch gekennzeichnet, daß sie pro Liter 11,17 mmol eines Na-Salzes des 3-Methoxy-4-(4'-aminobenzolsulfonamido)-1,2,5-Thiadiazol, 2,20 mmol 2,4-Diamino-5-(3',4',5'-trimethoxybenzyl)-pyrimidin, 140-180 Milliäquivalente L-Äpfelsäure, L-Serin oder L-Threonin oder die gleiche äquivalente Menge an Gemischen derselben sowie die zur Einstellung eines pH-Wertes von 6,5 bis 7,0 erforderliche Menge an NaOH, gegebenenfalls neben inerten Zusätzen zur Einstellung der Osmolarität auf physiologisch tolerierbaren Werte, enthält.

10. Verfahren zur Herstellung der intravenös verabreichbaren, wäßrigen Lösung gemäß den Ansprüchen 1 bis 9, dadurch gekennzeichnet, daß einer Lösung des Monosalzes des 3-Methoxy-4-(4'-aminobenzolosulfonamido)-1,2,5-thiadiazols mit einer starken, physiologisch tolerierbaren Base in Wasser Äpfelsäure zusammen mit etwa der zweifach molaren Menge einer starken, physiologisch tolerierbaren Base, L-Serin und/oder L-Threonin als innere Salze unter Bildung einer klaren, wäßrigen Lösung zugefügt, in diese Lösung das 2,4-Diamino-5-(3',4',5'-trimethoxybenzyl)-pyrimidin eingetragen und bei Temperaturen von Raumtemperatur bis etwa 90° C in Lösung gebracht wird, worauf der pH-Wert der resultierenden Lösung durch Zugabe der starken Base oder von Äpfelsäure auf den gewünschten Wert im Bereich von 6,4 bis 7,2 eingestellt und nötigenfalls die Osmolarität durch Zugabe inerter, niedermolekularen Substanzen auf einen physiologische tragbaren Wert gebracht wird.

## Claims

1. Aqueous, intravenously administerable solution of the sulfonamide 3-methoxy-4-(4'-aminobenzenesulfonamido)-1,2,5-thiadiazole present as a salt and of 2,4-diamino-5-(3',4',5'-trimethoxybenzyl)-pyrimidine, characterized in that it comprises malic acid, L-serine and/or L-threonine as a solubilizer for the 2,4-diamino-5-(3', 4', 5'-trimethoxybenzyl)-pyrimidine together with a strong, physiologically tolerable base forming water soluble salts with the sulfonamide and the malic acid in an amount equimolar to the amount of the sulfonamide present as well as in an amount of approximately 2 mol per mol of malic acid present, wherein the pH value of the solution is adjusted to the range 6.4-7.2.

2. Solution according to claim 1, characterized in that the acids serving as a solubilizer are present in an amount of at least 100 milliequivalents per liter solution.

3. Solution according to the claims 1 or 2, characterized in that the acids serving as a solubilizer are present in an amount of 120-180 milliequivalents per liter solution.

4. Solution according to the claims 1 to 3, characterized in that the pH value is adjusted to the range of 6.5-7.0.

5. Solution according to the claims 1 to 4, characterized in that the solubilizer is malic acid and the strong base is NaOH or a mixture of NaOH and KOH, wherein the concentration of KOH does not exceed 25 mmol/l of the solution.

6. Solution according to the claims 1 to 4, characterized in that the solubilizer is L-serine and/or L-threonine and the strong base is NaOH and/or KOH.

7. Solution according to the claims 1 to 6, characterized in that it possesses an osmolarity of at least 200 mosmol/l.

8. Solution according to the claims 1 to 7, characterized in that the solubilizer is L-malic acid in salt form.

9. Solution with anti-bacterial action according to the claims 1 to 8, characterized in that it comprises per liter 11.17 mmol of a Na salt of 3-methoxy-4-(4'-aminobenzenesulfonamido)-1,2,5-thiadiazole, 2.20 mmol 2,4-diamino-5-(3',4',5'-trimethoxybenzyl)-pyrimidine, 140-180 milliequivalents L-malic acid, L-serine or L-threonine or the same equivalent amount of mixtures of the same as well as the required amount of NaOH for the adjustment of a pH value of 6.5 to 7.0, optionally in addition to inert additives for the adjustment of the osmolarity to physiologically tolerable values.

10. Method for the production of the aqueous, intravenously administerable solution according to the claims 1 to 9, characterized in that malic acid together with approximately the two-fold molar amount of a strong, physiologically tolerable base, L-serine and/or L-threonine as internal salts is added to a solution of the monosalt of 3-methoxy-4-(4'-aminobenzenesulfonamido)-1,2,5-thiadiazole with a strong, physiologically tolerable base in water under formation of a clear, aqueous solution, 2,4-diamino-5-(3',4',5'-trimethoxybenzyl)-pyrimidine is introduced into this solution and brought into solution at temperatures from room temperature to approximately 90°C, whereupon the pH value of the resulting solution is adjusted to the desired value in the range of 6.4 to 7.2 by addition of the strong base or of malic acid and, if necessary, the osmolarity is brought to a physiologically acceptable value by addition of inert, low molecular substances.

## Revendications

1. Solution aqueuse administrable par voie intraveineuse du sulfonamide 3-méthoxy-4-(4'-aminobenzènesulfonamido)-1,2,5-thiadiazole présent sous forme de sel et de 2,4-diamino-5-(3',4',5'-triméthoxybenzyl)-pyrimidine, caractérisée en ce qu'elle contient comme tiers solvant pour la 2,4-diamino-5-(3',4',5'-triméthoxybenzyl)-pyrimidine l'acide malique, la L-sérine et/ou la L-thréonine, avec une base forte, physiologiquement tolérable, formant avec le sulfonamide et l'acide malique des sels solubles dans l'eau en une quantité équimolaire à la quantité de sulfonamide présent ainsi qu'en une quantité d'environ 2 moles par mole d'acide malique présent, le pH de la solution étant réglé dans un intervalle de 6,4-7,2.

2. Solution selon la revendication 1, caractérisée en ce que les acides servant de tiers solvants sont ajoutés en une quantité d'au moins 100 milliéquivalents par litre de solution.

3. Solution selon les revendications 1 ou 2, caractérisée en ce que les acides servant de tiers solvant sont ajoutés en une quantité de 120-180 milliéquivalents par litre de solution.

4. Solution selon les revendications 1 à 3, caractérisée en ce que le pH est réglé dans un intervalle de 6,5-7,0.

5. Solution selon les revendications 1 à 4, caractérisée en ce que le tiers solvant est l'acide malique et la base forte NaOH ou un mélange de NaOH et de KOH, la concentration en KOH ne dépassant pas 25 mmol/l de la solution.

6. Solution selon les revendications 1 à 4, caractérisée en ce que le tiers solvant est la L-sérine et/ou la L-thréonine et en ce que la base forte est NaOH et/ou KOH.

7. Solution selon les revendications 1 à 6, caractérisée en ce qu'elle possède une osmolarité d'au moins 200 mosmol/l.

8. Solution selon les revendications 1 à 7, caractérisée en ce que le tiers solvant est l'acide L-malique sous forme de sel.

9. Solution à action antibactérienne selon les revendications 1 à 8, caractérisée en ce qu'elle contient, par litre, 11,17 mmol d'un sel de Na de 3-méthoxy-4-(4'-aminobenzènesulfonamido)-1,2,5-thiadiazole, 2,20 mmol de 2,4-diamino-5-(3',4',5'-triméthoxybenzyl)-pyrimidine, 140-180 milliéquivalents d'acide L-malique, de L-sérine ou de L-thréonine ou la même quantité équivalente de mélange de ces corps ainsi que, pour établir une valeur de pH de 6,5 à 7,0 , la quantité nécessaire de NaOH, outre le cas échéant des additifs inertes pour régler l'osmolarité à des valeurs physiologiquement tolérables.

10. Procédé de préparation de la solution aqueuse, administrable par voie intraveineuse, selon les revendications 1 à 9, caractérisé en ce qu'à une solution du monosel de 3-méthoxy-4-(4'-aminobenzènesulfonamido)-1,2,5-thiadiazole avec une base forte physiologiquement tolérable dans l'eau on ajoute de l'acide malique avec une quantité environ deux fois molaire d'une base forte physiologiquement tolérable, de L-sérine et/ou de L-thréonine sous forme de sels internes avec formation d'une solution aqueuse claire, en ce qu'on introduit dans la solution la 2,4-diamino-5-(3',4',5'-triméthoxybenzyl)-pyrimidine et en ce qu'on l'amène en solution à des températures allant de la température ambiante à environ 90°C, avec réglage du pH de la solution résultante par addition de la base forte ou d'acide malique à la valeur désirée dans un intervalle de 6,4 à 7,2, et si nécessaire en ce qu'on amène l'osmolarité à une valeur physiologiquement acceptable par addition de substances inertes de faible poids moléculaire.
